Europäisches Patentamt

European Patent Office

Office européen des brevets

(19)

(11) Publication number: **0 136 100 B1**

(12) **EUROPEAN PATENT SPECIFICATION**

(45) Date of publication of patent specification: **29.07.92**  (51) Int. Cl.⁵: **A61K 31/70, A61K 47/00**

(21) Application number: **84305891.8**

(22) Date of filing: **29.08.84**

(54) Sucralfate suspension for use in treating ulcers.

(30) Priority: **02.09.83 US 528976**

(43) Date of publication of application:
**03.04.85 Bulletin 85/14**

(45) Publication of the grant of the patent:
**29.07.92 Bulletin 92/31**

(84) Designated Contracting States:
**AT BE CH DE FR GB IT LI LU NL SE**

(56) References cited:
**EP-A- 133 880**
**EP-A-01 072 09**
**DE-A- 3 322 078**
**DE-B- 1 568 346**
**US-A- 3 432 489**

**ARZNEIMITTELFORSCHUNG, vol. 29(II), no. 11, November 1979, pages 1668-1676, Aulendorf, DE; R. NAGASHIMA et al.: "Sucralfate, a basic aluminum salt of sucrose sulfate"**

**H.P. FIEDLER: "Der Pharmazeutische Betrieb", vol. 9, 2nd edition, 1981, pages 887,888, Editio Cantor, Aulendorf, DE; "Lexikon der Hilfsstoffe für Pharmazie, Kosmetik und angrenzende Gebiete"**

(73) Proprietor: **Marion Merrell Dow Inc.**
**10236 Bunker Ridge Road**
**Kansas City Missouri 64137(US)**

(72) Inventor: **Casillan, Angel B.**
**12101 W. 103rd Terrace**
**Overland Park, KS 66215(US)**
Inventor: **Frazier, William F.**
**9908 Nieman Place**
**Overland Park, KS 66204(US)**

(74) Representative: **Myerscough, Philip Boyd et al**
**J.A.Kemp & Co. 14, South Square Gray's Inn**
**London, WC1R 5EU(GB)**

EP 0 136 100 B1

Scandinavian Journal of Gastroenterology March 1983, 15-16;

Biomedical Information 1979, 307-320;

Abstracts of the X1 International Congress of Gastoenterology, 1980;

Journal of Clinical Gastroenterology 1981, 3 (suppl. 2), 103-110;

Textbook of Pharmacology, Guildford, 1980, 25.II;

Nagashima R. and Yoshida N., Arzneimittelforschung-Drug Research 29(II), Nr. 11 (1979) pp. 1668-1676;

Physical Pharmacy, 2nd Edition Martin, Swarbrick, Cammarata Lea & Febiger 1973, pages 444, 451, 516, 517, 520;

The Theory and Practice of Industrial Pharmacy Lachman, Lieberman, Kanig Lea & Febiger 1970, pages 517, 518, 527-530; U.S. Pharmacopeia, USP XX, 1980, page 1030;

U.S. Pharmacopeia, USP XX, 1980, page 1030;

Galenisches Prakticum, Münzel, Büchi, Schultz, 1959, page 409.

**Description**

Sucralfate is a disaccharide polysulfate aluminum compound useful in the treatment of duodenal ulcers. This invention provides the use of sucralfate in the preparation of a pharmaceutical suspension for use in the treatment by oral administration of ulcerative conditions of the esophagus.

Sucralfate is a disaccharide polysulfate aluminum compound which is described in US-A-3,432,489. Sucralfate is believed to be 3,4,5,6-tetra-(polyhydroxyaluminum)-alpha-D-glycopyranosyl sulfate-2,3,4,5-tetra-(polyhydroxyaluminum)-beta-D-fructofuranoside sulfate.

Sucralfate is useful in the treatment of ulcers in the duodenum. Sucralfate is available in tablets which disintegrate rapidly in the stomach releasing the sucralfate for reaction with ulcer tissues in the duodenum.

To date, sucralfate has not been available in a form which would render it effective in treatment of ulcerative conditions of the esophagus. The difficulty in formulating a sucralfate composition for such treatment has characteristically been due to the inability to supply sucralfate in a form which would be available in the esophagus. Formulations, including liquid formulations, have been tried without success.

It has now been discovered that sulcralfate can be made available for treatment of ulcer tissues in the esophagus when formulated as a suspension which forms a sticky gel-like or tacky viscous mass which settles to the bottom of a reaction vessel when added to 0.2 to 1.0 N hydrochloric acid.

According to the invention there is provided the use of a disaccharide polysulfate aluminum compound in the preparation of a pharmaceutical suspension for use in the treatment by oral administration of ulcerative conditions of the esophagus, the suspension comprising a pharmaceutically effective amount of a disaccharide polysulfate-aluminum compound containing 7-13% sulfur and 11-24% aluminum per molecule and a pharmaceutically acceptable suspending agent in an aqueous suspension, said suspension being capable of forming a viscous mass when added to 0.2 to 1.0 N hydrochloric acid, particularly 0.5 N hydrochloric acid.

The suspension may additionally contain bodying agents, preservatives, antifoam agents, flavors and coloring agents.

The invention will be described with specific reference to sucralfate. However, it should be understood that suspensions of other ulcer treating agents disclosed in US-A-3,432,489 can be used in accordance with the present invention. That is, the use of any disaccharide polysulfate-aluminum compound containing 7-13% sulfur and 11-24% aluminum per molecule is within the scope of the present invention.

Preferably the composition is a suspension of sucralfate. The suspension is in a form which ensures that sucralfate is available to react with ulcer tissues in the esophagus with which it comes in contact after oral administration, in contrast to prior art sucralfate formulations in which sucralfate is not released until it reaches the stomach and duodenum.

The only limitations on the amounts and types of suspending agents useful in the practice of the invention are 1) that they be pharmaceutically acceptable and 2) that when used with sucralfate, the resulting suspension forms a tacky viscous mass, which settles at least partially to the bottom of the reaction vessel, upon addition to 0.2 to 1 N hydrochloric acid. If, upon addition to the hydrochloric acid the particles remain suspended or there is no reaction, the suspension is not acceptable for the purposes of the present invention. The significance of the formation of the sticky mass is not fully understood. However, formulations of sucralfate which have this characteristic provide sucralfate to any ulcer tissue with which the suspension comes in contact after administration; that is the sucralfate is available for reaction with ulcer tissues as soon as it passes through the oral cavity.

The sucralfate suspension can be formulated from a wide range of suspending agents, including but not limited to gums, celluloses, clays and silicas and certain polymers, particularly vinyl or acrylic polymers. The following are representative of pharmaceutically acceptable suspending agents which can be employed in this invention: acacia, agar, alginic acid, carrageenan, guar, pectin, tragacanth, xanthan, carboxymethylcellulose sodium, hydroxypropyl cellulose, hydroxypropyl methylcellulose, methylcellulose, microcrystalline cellulose, aluminum magnesium silicate, bentonite, hectorite, kaolin, colloidal silicon dioxide, polyacrylic acid, polyvinyl alcohol, and polyvinylpyrrolidone polymers.

The suspension may contain suspension adjuvants to improve patient acceptance and quality of the product. Ingredients such as (but not limited to) glycerin, sorbitol, glucose and sugar, can be added to increase the body of the suspension. Preservatives such as (but not limited to) benzalkonium chloride, benzyl alcohol, parahydroxybenzoic acid esters and sorbic acid and its salts can be added to suppress growth of microorganisms. Antifoam agents such as the silicones can be added to dissipate foam produced when the product is shaken prior to use. Finally, flavors and color may be added to improve palatability.

In preferred practice, sucralfate is formulated with microcrystalline cellulose, colloidal silicon dioxide and/or methyl cellulose as the suspending agent. The preferred formulation and variations thereof which are

3

acceptable for purposes of the present invention (i.e. bring about the appropriate acid reaction) are as follows:

| INGREDIENT | CONC. % w/v | ALLOWABLE RANGES, % W/V |
|---|---|---|
| Sucralfate | 10.00 | 1 to 60 |
| Microcrystalline Cellulose NF | 1.00 | 0 to 7 |
| Colloidal Silicon Dioxide NF | 0.20 | 0 to 5 |
| Methylcellulose USP, 4 Pas | 0.25 | 0 to 1 |
| Sorbitol 70% | 20.00 | 0 to 60 |
| Methylparaben | 0.20 | * |
| Glycerin 99.5% | 9.90 | 0 to 40 |
| Simethicone 30% Emulsion | 0.30 | * |
| Water DI Filt. | Q.S. | |
| Total: | 100.0 | |
| * Q.S. to effective level. | | |

In the above formulations, the sorbitol and glycerin are bodying agents; the methylparaben is a preservative; and the simethicone operates as an antifoaming agent. The microcrystalline cellulose, silicon dioxide and methylcellulose are the suspending agents.

The following Example is illustrative of the invention. Unless the context requires otherwise, all percentages in the Example and elsewhere in this specification are weight percentages/volume.

Example

The following compositions, which form a tacky viscous mass when added to 0.5 N hydrochloric acid, are within the invention:

| | | % W/V | | |
|---|---|---|---|---|
| INGREDIENT | | A | B | C |
| Sucralfate | | 10.00 | 10.00 | 10.00 |
| Methylcellulose (0.4 Pas) | | 0.75 | | |
| Methylcellulose (4 Pas) | | | | 1.50 |
| Colloidal silicon dioxide | | | 0.25 | 0.20 |
| Microcrystalline cellulose | | | 1.00 | 1.00 |
| Aluminum magnesium silicate | | 1.00 | | |
| Methylparaben | | 0.12 | 0.15 | 0.15 |
| Propylparaben | | 0.08 | | |
| Glycerin | | | 9.90 | 9.90 |
| Sorbitol 70% | | 40.00 | 20.00 | 20.00 |
| Simethicone 30% | | | | 0.30 |
| Water | | Q.S. | Q.S. | Q.S. |
| Total | | 100 | 100 | 100 |

## Claims

1. The use of a disaccharide polysulfate aluminum compound in the preparation of a pharmaceutical suspension for use in the treatment by oral administration of ulcerative conditions of the esophagus, the suspension comprising a pharmaceutically effective amount of a disaccharide polysulfate-aluminum compound containing 7-13% sulfur and 11-24% aluminum per molecule and a pharmaceutically acceptable suspending agent in an aqueous suspension, said suspension being capable of forming a viscous mass when added to 0.2 to 1.0 N hydrochloric acid.

2. Use according to claim 1 wherein the disaccharide compound is sucralfate.

3. Use according to claim 1 or 2 wherein the suspending agent is at least one gum, cellulose, clay, silica or vinyl or acrylic polymer.

4. Use according to claim 3 wherein the suspending agent is selected from acacia, agar, alginic acid, carageenan, guar, pectin, tragacanth and xanthan.

5. Use according to claim 1 wherein sucralfate is formulated with at least one of microcrystalline cellulose, colloidal silicon dioxide and methyl cellulose as suspending agent.

6. Use according to claim 5 wherein the suspension is an aqueous suspension containing 10% w/v sucralfate and employing a suspending agent composed of 1% w/v microcrystalline cellulose, 0.2% w/v colloidal silicon dioxide and 0.25% w/v methylcellulose (4 Pas), in combination with 20% w/v sorbitol (70%) and 9,9% w/v glycerin (99.5%) as bodying agents, 0.2% w/v methylparaben as a preservative and 0.3% simethicone (30% emulsion) as an antifoaming agent.

**Revendications**

1. Utilisation d'un composé de disaccharide polysulfate d'aluminium dans la fabrication d'une suspension pharmaceutique pour son utilisation dans le traitement par administration orale de conditions ulcératives de l'oesophage, la suspension comprenant une quantité pharmaceutiquement efficace d'un composé de disaccharide polysulfate d'aluminium contenant 7-13% de soufre et 11-24% d'aluminium par molécule et un agent de suspension pharmaceutiquement acceptable dans une suspension acqueuse, ladite suspension étant capable de former une masse visqueuse quand elle est ajoutée à de l'acide chlorhydrique 0,2 à 1,0N.

2. Utilisation selon la revendication 1 dans laquelle le composé de disaccharide est le sucralfate.

3. Utilisation selon la revendication 1 ou 2 dans laquelle l'agent de suspension est au moins une gomme, la cellulose, une argile, la silice ou un polymère vinylique ou acrylique.

4. Utilisation selon la revendication 3 dans laquelle l'agent de suspension est choisi parmi la gomme arabique, l'agar, l'acide aiginique, le carageenan, la gomme de guar, la pectine, la gomme adragante et la gomme xanthane.

5. Utilisation selon la revendication 1 dans laquelle le sucralfate est formulé avec au moins un composé choisi parmi la cellulose microcristalline, le dioxyde de silicium colloïdal et la méthylcellulose comme agent de suspension.

6. Utilisation selon la revendication 5 dans laquelle la suspension est une suspension acqueuse contenant 10% p/v de sucralfate et un agent de suspension composé de 1% p/v de cellulose microcristalline, 0,2% p/v de dioxyde de silicium colloïdal et 0,25% p/v de méthylcellulose (4 Pa.s), en combinaison avec 20% p/v de sorbitol (70%) et 9,9% p/v de glycérine (99,5%) comme agents de texture, 0,2% p/v de méthylparaben comme conservateur et 0,3% de simethicone (émulsion à 30%) comme agent antimousse.

**Patentansprüche**

1. Verwendung einer Disaccharidpolysulfat-Aluminiumver-bindung zur Herstellung einer pharmazeutischen Suspension zur Anwendung bei der Behandlung, durch orale Verabreichung, von Geschwürzuständen des Esophagus, wobei die Suspension eine pharmazeutisch wirksame Menge einer Disaccharidpolysulfat-Aluminiumverbindung mit 7 bis 13 % Schwefel und 11 bis 24 % Aluminium pro Molekül und ein pharmazeutisch annehmbares Suspendiermittel in einer wäßrigen Suspension umfaßt und wobei die Suspension eine viskose Masse bilden kann, wenn sie in 0,2 bis 1,0 N Salzsäure gegeben wird.

2. Verwendung nach Anspruch 1, worin die Disaccharidverbindung Sucralfat ist.

3. Verwendung nach Anspruch 1 oder 2, worin das Suspendiermittel wenigstens ein Gummi, eine Zellulose, eine Tonerde, ein Kieselgel oder ein Vinyl- oder Acrylpolymer ist.

4. Verwendung nach Anspruch 3, worin das Suspendiermittel aus Akaziengummi, Agar, Alginsäure, Carrageen, Guar, Pectin, Traganth und Xanthan ausgewählt ist.

5. Verwendung nach Anspruch 1, worin Sucralfat mit wenigstens einem aus mikrokristalliner Zellulose, kolloidalem Siliciumdioxid und Methylzellulose als Suspendiermittel formuliert ist.

6. Verwendung nach Anspruch 5, worin die Suspension eine wäßrige Suspension mit 10 % G/V Sucralfat unter Verwendung eines aus 1 % G/V mikrokristalliner Zellulose, 0,2 % G/V kolloidalem Siliciumdioxid und 0,25 % G/V Methylzellulose (4 Pas) zusammengesetzten Suspendiermittels in Kombination mit 20 % G/V Sorbit (70 %) und 9,9 % G/V Glycerin (99,5 %) als Körperbildungsmittel, 0,2 % G/V Methylparaben als Konservierungsmittel und 0,3 Simethicon (30 %ige Emulsion) als Antischaummittel ist.